# EUROPEAN PATENT APPLICATION

(11) **EP 2 492 894 A1**
(43) Date of publication of application: **29.08.2012**
(21) Application number: 11425043.4
(22) Date of filing: 22.02.2011
(51) Int. Cl.: G09B 23/28, A61N 1/08, G06F 19/00

(54) **System for remote monitoring of health conditions and for the administration of therapies**

(71) Applicant: Fiorino, Giuseppe, 70020 Cassano delle Murge (BA) (IT)
(72) Inventor: Fiorino, Giuseppe, 70020 Cassano delle Murge Bari (IT); Piccininno, Giovanni, 70024 Gravina in Puglia Bari (IT)
(74) Representative: Bruni, Giovanni

(57) **Abstract**

Device for remote monitoring the health state of a patient by a health care professional by means of audio and video communications means. The device is provided with a supervision unit, an internet connection means, sensors and respective printed circuit boards for monitoring the physiologic parameters of the patient, equipments for therapy administration to the patient and a PLC able to control the printed circuit boards and the equipments for the therapy administration. The supervision unit and the PLC allow to perform simultaneously the activation or deactivation operations of said equipments for therapy administration to the patient, to remotely monitor in real time the physiological parameters of the patient sensed by the sensors, and to perform video-calls. The data sensed can be used to evaluate instantly the efficacy of the therapy administration.

## Description

The present invention relates to a web-based system for remote monitoring the health state of a patient.

According to the state of the art, there are known various devices allowing to facilitate the control of home assistance to patients suffering from chronic pathologies.

The United State patent US2008011923 "Method for establishing a telecommunications network for patient monitoring" describes for example a wireless peer-to-peer configured network, which is also provided with two kinds of nodes of which the first one can be a wireless peripheral able to monitor the physiologic parameters of a patient, the second one is made up of wireless peripherals able to receive data if they are in the range of the wireless transmitter.

The French patent FR0100234 describes a system for patient's home assistance made up of an interface at the patient and an interface at the doctor, which are connected by means of the telephone system to a server which receives from the interface arranged at patient's home the values of the physiologic parameters measured by the patient and is able to store the time history of such values in determined time intervals. On the server there are also provided data processing means which determine, in function of the patient's history and the physiologic parameters, the probability model for the different physiologic conditions of the patient.

Among the devices known according to the state of the art and available on the market, there are devices which allow to remotely monitor various physiologic parameters of the patient, such for example the blood pressure at regular time intervals, which are pre-set by the health care professional. At the state of the art, there are also known various software solutions which, upon installation of a specific application on the computer, allow the health care professionals to display and analyze the personal data of patients saved in a remote server.

Another type of device available on the market is a device which can be worn by the patient, which allows to sense the various physiologic parameters during his normal life, and to send by means of a mobile telephone connection the acquired data to a remote server for a following analysis by the health care professionals.

These and other applications yet known at the state of the art are limited, since they do not allow to perform simultaneously the remote activation operations of therapy administration, for example the oxygen administration, to video-communicate with the patient, to receive instantly the sensed data from the vital parameters monitor. The simultaneity of the enlisted operations would allow the health care professionals to use the information obtained directly by the patient by means of vide-call and from the data acquired in real time by the vital parameters monitor as feed back to verify the efficacy of the remote-activated therapy administration.

The applications known at the state of the art, in order to access to the health data of patients, need dedicated software installed on devices of different kind, such as for example desktop PC, portable computers, palmtops or the like. It is impossible to access to such information by means of a device provided with browser web but not provided with dedicated software.

Aim of the present invention is to provide a device able to perform simultaneously and remotely the activation operations of therapy administration, of video-communication with the patient, of receiving in real-time data sensed by the system for monitoring the vital parameters, thus allowing the health care professional to use the information obtained directly from the patient by means of video-call and from the data sensed in real-time by the vital parameters monitor as feed back to verify the efficacy of the remote-activated therapy administration.

More generally, the present invention describes a device able to reproduce completely the hospital visits at patient's home and to allow to monitor the patient's vital parameters.

According to another aspect, the present invention is a device able to allow remote-operating health care professionals to access to the patient's physiologic parameters sensed in real time by the monitoring system of vital parameters and to the information contained in the patient's medical record by means of any device provided with browser web, as for example a desktop PC, a portable PC, a tablet PC or a smart-phone, without the need of installing a dedicated software on this device.

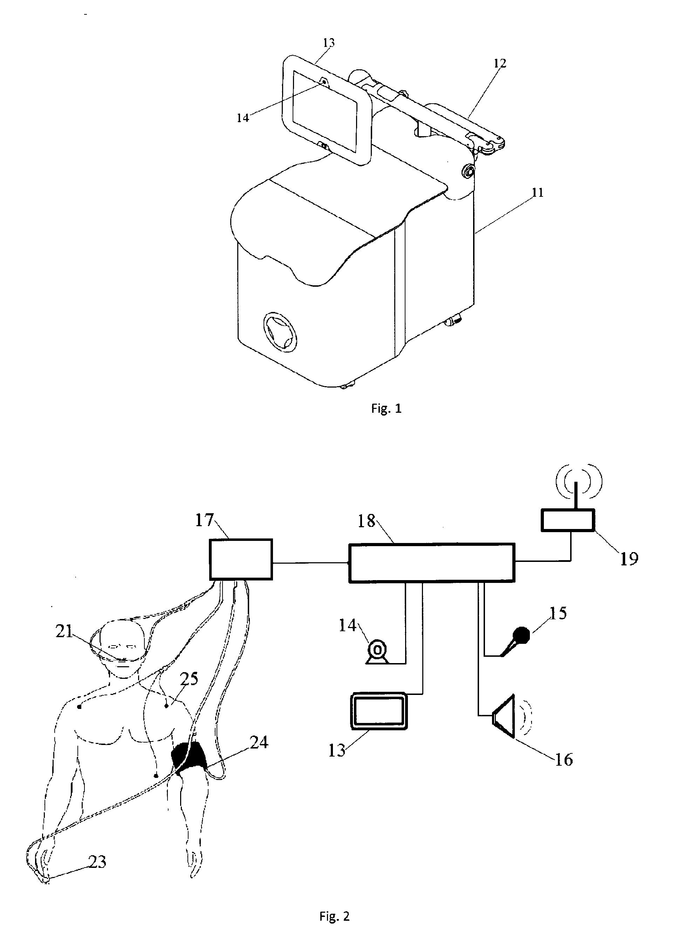

The present invention solves the above-described aims since it is a transportable and installable at patient's home system, provided with sensors for monitoring patient's physiologic parameters, therapy administration systems, such for example medical oxygen, and an audio-video communication system. More examples of the invention can be connected by means of a web portal with a remote station where a health care professional can monitor the patient's physiologic parameters, activate or deactivate the administration of various kinds of therapies and, if it is the case, share the data concerning the patient's medical record with a consultant (for example a specialist) via web, by means of a portal accessible from any device provided with browser web, in a simple way and respecting the privacy of the same patient. These and other advantages will be highlighted in the following detailed description referring to figures 1 to 6.

Figure 1 shows an embodiment of the device according to the present invention;
Figure 2 shows a functioning scheme of the device according to the present invention;
Figure 3 shows a scheme of the informatics infrastructure serving the device according to present invention;
Figure 4 shows another scheme of the web network allowing to use simultaneously more devices according to the present invention.

Figures 5 and 6 show two example images of the positions in which it is possible to use the device according to the present invention.

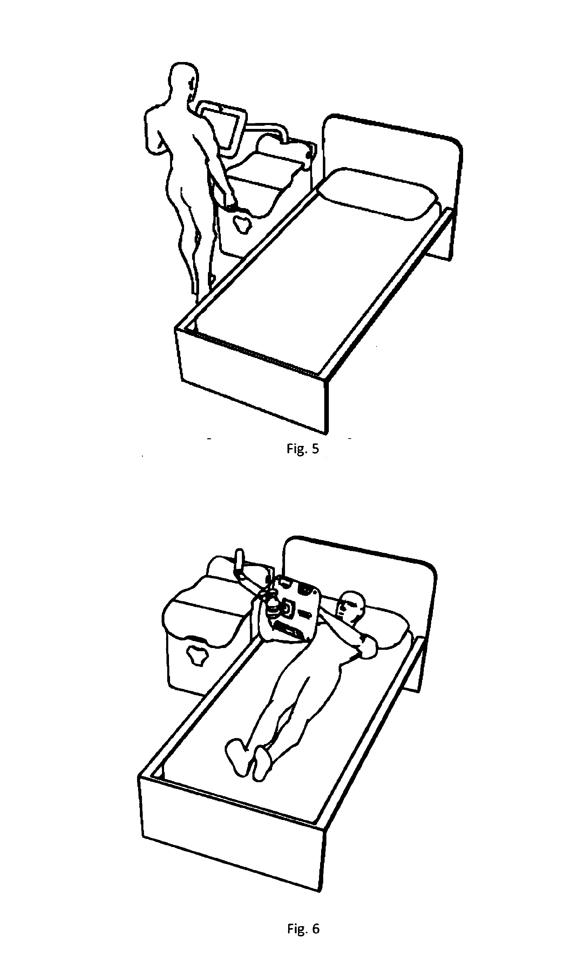

As it is shown in figure 1, the device according to the present invention is made up of a container which in an embodiment can be shaped as a bedside table (11) to which it is connected a monitor (13). The monitor (13) can be connected to the bedside table (11) by means of a articulated arm (12). As it is shown in figures 5 and 6 the articulated arm (12) is optimized for a comfortable use both by the patient lying on his back or sat and by a standing health care professional.

Inside the bedside table (11) there are contained equipments needed for the device functioning. As it is schematized in figure 2, there are in fact provided an oxygen concentrator (21) and a vacuum machine, controlled by means of a PLC (17) to which there are connected also printed circuit boards (22) for sensing vital parameters. To these last, there are connected sensors (23, 24, 25, 26) monitoring the physiologic parameters.

The remote monitoring of a patient at his own home implies in fact the control of vital parameters representing the patient's health state. To do so, it is needed to bring at the patient's home all the equipments needed for monitoring his physical state, and all the technologies needed to verify the results of the applied therapies and to control electro-medical devices available on the market, for example the medicinal gas supplying or the vacuum application.

All these equipments have to be efficient in monitoring the patient's health state and easy to be installed and used at patient's home as well. According to a first embodiment of the present invention the sensors are of the wired type, as those ones commonly used in hospital, so that the recharging problems are avoided and the patient has the sensation that the device being used is similar to the one used in hospital.

According to another embodiment of the present invention, the sensors for the patient's physiologic parameters can be of non-wired type and can communicate with the device by means of any technology able to exchange data in absence of a physical connection.

An oxygen concentrator can be integrated in the device. The integration of an oxygen concentrator in a device installable at patient's home allows to avoid the logistics problems linked to the use of medical oxygen cylinders. It is no more possible in fact that the patient remains without oxygen when it is not possible to supply a spare cylinder to him in rapid times. Besides the safety for the patient concerning the availability of oxygen, it is obtained the advantage that the health structures do not have to control the logistic linked to the distribution and the collection of the medical oxygen cylinders.

The interpretation of the signals from the sensors (23, 24, 25, 26) and the activation of the therapy devices (21) physically-controlled by PLC (17) can be controlled by a health care professional, who is remote-operating via web. As it is shown in fig. 2, in fact PLC communicates with an industrial PC (18) which controls a web-cam (14) integrated in the touch screen monitor (13) installed on the articulated arm (12), besides an audio system comprising loudspeakers (16) and microphone (14). The access of the device to the network occurs by means of a wired or wireless technology which allows the web access. By way of example, in fig. 2 it is shown the use of a router 3G (19). Thanks to the internet connection, the medical devices (which for example can be made up of an oxygen concentrator and a vacuum machine) can be also remotely activated without needing the patient's intervention.

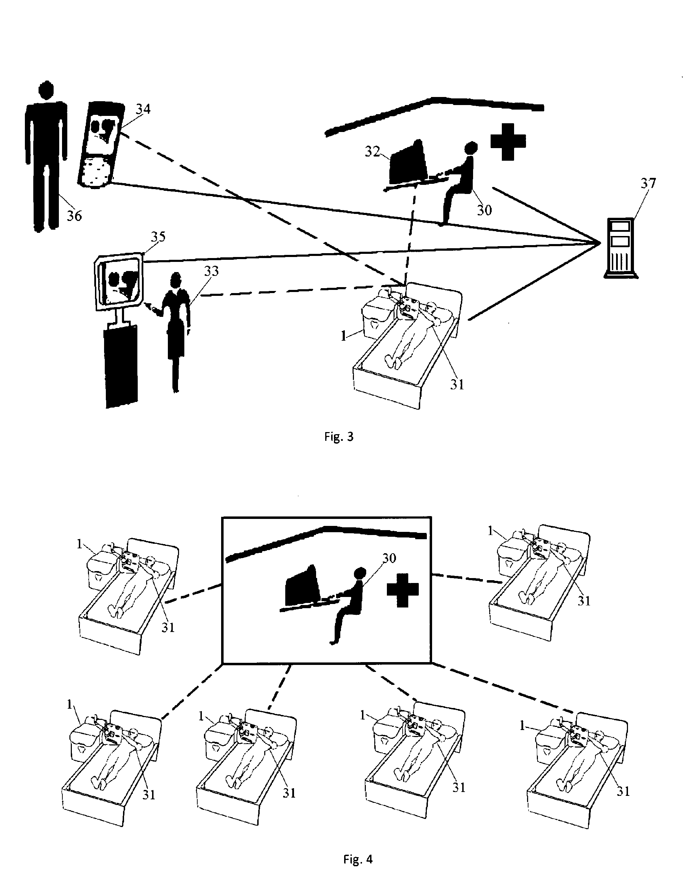

By means of the network, whose functional scheme is shown in fig. 3, a remote health care professional (30) can monitor the patient (31) 24 hours a day, since he has at his disposal the data concerning the patient's physiological parameters sensed by the sensors (23, 24, 25, 26) and can perform video-call sessions with the patient by means of the web-cam (14), the touch screen monitor (13), the loudspeakers (16) and the microphone (15). The possibility of a visual contact besides the vocal one with the patient allows to give the patient a better human relation with the health care professional, and the health care professional the possibility to visually monitor the onset of cognitive problems which are symptoms of disorders such for example dementia, Alzheimer and others. The use of the device by means of a web connection allows to put the patient in communication with other health care professionals (33, 36) who have access to the same network, such for example the family doctor or a specialist called for a consult. It is convenient to underline that, unlike various devices available on the market, the integration of web connectivity in the device allows to access to the network, upon authentication, with any device provided with web connectivity, such as for example a PC, a tablet or a mobile phone. Similarly, by using the authentication keys having limited time duration, it is possible for the specialist and the family doctor to access to clinical data of the patient without needing to bring along the dedicated device, but simply by accessing to the web portal of the home assistance network.

All the pieces of information concerning the communication sessions of all the actors of the home assistance network (patient (31), remote health care professional (30), family doctor, specialist (33, 36)), are saved for back-up in the remote server (37).

The preceding can be obviously extended to the case, shown in fig. 4, which provides a high number of patients (31) at whose home the present invention (1) is installed. Via web, a health care professional (30) can monitor the health state of a series of patients (31), each residing at his own home, by performing a "visit round" similar to the one performed at the hospital. Moreover, if needed, the health care professional (30) can contact other health care professionals (33, 36) who can communicate with patients from their own PC (35) or from their own palm-top (34) to evaluate the health conditions of the patient (31) also by displaying the data provided by the vital parameters monitor. Moreover, the provision of a touch-screen monitor (13) connected to a PC (18) allows to integrate in the device the possibility to perform exercises to practice the logical abilities or to use the entertaining applications as for example watching a film or listening to the music.

## Claims

1. Device for remote monitoring the health state of a patient by a health care professional, comprising audio (15, 16) and video (13, 14) communications means, a supervision unit (18), an internet connection means (19), sensors (23, 24, 25, 26) and respective printed circuit boards (22) for monitoring the physiologic parameters of the patient, equipments for therapy administration (12) to the patient and a PLC (17) able to control said printed circuit boards (22) and said equipments for the therapy administration (21), **characterized in that** said supervision unit (18) and said PLC (17) allow to perform simultaneously the activation or deactivation operations of said equipments for therapy administration to the patient (21), to remotely monitor in real time the physiological parameters of the patient sensed by said sensors (23, 24, 25, 26), and to perform video-calls by means of said audio (15, 16) and video (13, 14) communication means, and **in that** said simultaneity allows to use said data sensed by said PLC (17) and by said printed circuit boards (22) to evaluate instantly the efficacy of the administration of said therapy administered to said patient.

2. Device according to claim 1, **characterized in that** said data sensed by means of said PLC (17) and said printed circuit boards (22) are provided in real time for the automatic supervision or by means of health care professionals (32) provided with suitable authentication key operating on any device provided with browser web.

3. Device according to any one of claims 1 to 2, **characterized in that** said supervision unit (18) supplies constantly said remote operating health care professional with the data sensed in real time by the PLC (17) and said printed circuit boards (22).

4. Device according to claim 1, **characterized in that** said supervision unit (18), the housing of said sensors (23, 24, 25, 26) for monitoring the patient's physiologic parameters and of said printed circuit boards (22), said equipments for the administration to the patient of therapies (12), and said PLC (17) are housed inside a sole casing, such shaped and dimensioned that it is easily transported and positioned at the patient's home, on which it is fixed an articulated arm (13) to which there are connected said audio (15, 16) and video (13, 14) communication means.

5. Device according to claim 2, **characterized in that** when said casing is positioned next to the patient's bed, said articulated arm (12) allows to direct said audio (15, 16) and video (13, 14) communication means so that it is possible their use both by the patient in the bed and by a standing health care professional or person sat, without moving said casing.

6. Device according to claim 1, **characterized in that** said network connection means (19) allows the connection to the network without using the fixed telephone system.

7. Device according to claim 1, **characterized in that** said equipments for administration of therapies (21) comprise a medical oxygen concentrator and/or a vacuum machine.

8. Device according to claim 1, **characterized in that** said video communication means (13, 14) are made up of a touch screen monitor and a web-cam.

9. Method for using the device according to any one of the preceding claims comprising the following steps:
- storing in a server (37) the data sensed by the device according to any one of the preceding claims
- remote monitoring the patient (31) by a health care professional (30) by controlling the physiologic parameters and video-call sessions
- remote activation of the devices for administration of therapies (12)
- via web sharing the physiologic parameters of the patient with a specialist (36) who accesses, upon authentication from any device provided with web browser (34), both to the data acquired in real time and the ones stored on the server (37);
- medical home visit by the remote operating specialist (36) comprising the analysis of the data of the physiologic parameters of the patient and a video-call session.

10. Method for remote controlling the clinical record of the patient and for reports production of the clinical examinations having legal value by means of devices provided with web browser, comprising the following steps:
- storing in a server (37) the data sensed by the device according to any one of the preceding claims;
- access of a health care professionals having a suitable authentication key operating by means of any device provided with browser web to the clinical examinations of the patient containing the data concerning the vital parameters of the patient sensed by the device according to claims 1, 2, 3, 4 and saved in said server (37);
- reporting said clinical examinations by said health care professional having said authentication key;
- digital signature of the report concerning said clinical examination by said health care professional;
- saving said report in said server.

## Amended claims

### Amended claims in accordance with Rule 137(2) EPC.

**1.** Device for remote monitoring the health state of a patient by a health care professional, comprising
container,
- audio (15, 16) and video (13, 14) communications means,
- a supervision unit (18),
- an internet connection means (19),
- sensors (23, 24, 25, 26) and respective printed circuit boards (22) for monitoring the physiologic parameters of the patient,
- equipments for therapy administration (12) to the patient, said equipment being located inside said container and being installable at patient's home
- and a PLC (17) able to control said printed circuit boards (22) and said equipments for the therapy administration (21),
- said supervision unit (18) and said PLC (17) allowing simultaneously to remotely monitor In real time the physiological parameters of the patient sensed by said sensors (23, 24, 25, 26), and to perform video-calls by means of said audio (15, 16) and video (13, 14) communication means,
**characterized in that**
said supervision unit (18) and said PLC (17) also allow to perform simultaneously the activation or deactivation operations of said equipments for therapy administration to the patient (21),
and **in that**
said simultaneity allows to use said data sensed by said PLC (17) and by said printed circuit boards (22) to evaluate instantly the efficacy of the administration of said therapy administered to said patient.

**2.** Device according to claim 1, **characterized in that** said data sensed by means of said PLC (17) and said printed circuit boards (22) are provided in real time for the automatic supervision or by means of health care professionals (32) provided with suitable authentication key operating on any device provided with browser web.

**3.** Device according to any one of claims 1 to 2, **characterized in that** said supervision unit (18) supplies constantly said remote operating health care professional with the data sensed in real time by the PLC (17) and said printed circuit boards (22).

**4.** Device according to claim 1, **characterized in that** said supervision unit (18), the housing of said sensors (23, 24, 25, 26) for monitoring the patient's physiologic parameters and of said printed circuit boards (22), said equipments for the administration to the patient of therapies (12), and said PLC (17) are housed inside a sole casing, such shaped and dimensioned that it is easily transported and positioned at the patient's home, on which it is fixed an articulated arm (13) to which there are connected said audio (15, 16) and video (13, 14) communication means.

**5.** Device according to claim 2, **characterized in that** when said casing is positioned next to the patient's bed, said articulated arm (12) allows to direct said audio (15, 16) and video (13, 14) communication means so that it is possible their use both by the patient in the bed and by a standing health care professional or person sat, without moving said casing.

**6.** , Device according to claim 1, **characterized in that** said network connection means (19) allows the connection to the network without using the fixed telephone system.

**7.** Device according to claim 1, **characterized in that** said equipments for administration of therapies (21) comprise a medical oxygen concentrator and/or a vacuum machine.

**8.** Device according to claim 1, **characterized in that** said video communication means (13, 14) are made up of a touch screen monitor and a web-cam,

**9.** Method for using the device according to any one of the preceding claims comprising the following steps:
- storing in a server (37) the data sensed by the device according to any one of the preceding claims
- remote monitoring the patient (31) by a health care professional (30) by controlling the physiologic parameters and video-call sessions
- remote activation of the devices for administration of therapies (12)
- via web sharing the physiologic parameters of the patient with a specialist (36) who accesses, upon authentication from any device provided with web browser (34), both to the data acquired in real time and the ones stored on the server (37);
- medical home visit by the remote operating specialist (36) comprising the analysis of the data of the physiologic parameters of the patient and a video-call session.
